# EUROPÄISCHE PATENTANMELDUNG

(11) **EP 2 886 153 A1**
(43) Veröffentlichungstag der Anmeldung: **24.06.2015**
(21) Anmeldenummer: 13199189.5
(22) Anmeldetag: 20.12.2013
(51) Int. Cl.: A61N 1/04, A61N 1/32, A61N 1/30, A61F 13/40

(54) **System für die transdermale Abgabe von Wirkstoff**

(71) Anmelder: LTS LOHMANN Therapie-Systeme AG, 56626 Andernach (DE)
(72) Erfinder: Sameti, Mohammad, 53177 Bonn (DE); Hackbarth, Ronald, 56075 Koblenz (DE); Schumann, Klaus, 56567 Neuwied (DE); Schmitz, Christoph, 56598 Rheinbrohl (DE)
(74) Vertreter: Schweitzer, Klaus

(57) **Zusammenfassung**

Die Anmeldung betrifft ein transdermales therapeutisches System (TTS) für die Abgabe von pharmazeutischen Wirkstoffen umfassend eine Deckschicht (7) und mindestens ein wirkstoffhaltiges Trägermaterial (1), wobei sich mindestens ein Halteelement (6a-6d) zwischen wirkstoffhaltigem Trägermaterial und Deckschicht befindet, mit dem das wirkstoffhaltige Trägermaterial an der Deckschicht fixiert ist. Die Anmeldung betrifft auch ein Verfahren zum Befestigen eines wirkstoffhaltigen Trägermaterials an einer Deckschicht eines TTS in Anwesenheit von Klettbandsegmenten und die Verwendung eines Klettbandes in einem Verfahren zur transdermalen oder iontophoretischen Verabreichung von pharmazeutischen oder therapeutischen Wirkstoffen an Patienten.

## Beschreibung

Die vorliegende Erfindung betrifft ein neues System für die Abgabe von pharmazeutischen Wirkstoffen in therapeutisch wirksamer Menge an den Organismus.

Vorzugsweise betrifft die vorliegende Erfindung ein einfach zu handhabendes System für die transdermale Abgabe von in Flüssigkeit gelöstem, pharmazeutischem Wirkstoff in therapeutisch wirksamer Menge, besonders bevorzugt die transdermale Abgabe von kationischem Wirkstoff mittels lontophorese.

Der transdermale Weg der parenteralen Verabreichung bietet zahlreiche Vorteile gegenüber anderen Verabreichungswegen. Verfahren und Systeme zur Verabreichung von Arzneimitteln durch die Haut sind auf dem Gebiet der Pharmazie weitgehend bekannt. Typischerweise erfolgt die transdermale Verabreichung mit passiven transdermalen Systemen (z.B. Transdermale, Therapeutische Systeme, TTS), bei denen pharmazeutische Wirkstoffe in definierten Mengen mittels Diffusion durch die Haut dem Organismus zugeführt werden.

Speziell bei der transdermalen Zufuhr von in Flüssigkeit gelösten Wirkstoffen gibt es das Problem, dass ein wirkstoffhaltiges Trägermaterial aus Vlies oder aus Schwammtuch oder ein Gel getrennt von der Deckschicht, mit der zusammen der Wirkstoff im TTS an der Haut fixiert wird, aufbewahrt werden muss, weil sonst die Lagerstabilität über eine längere Zeitdauer hinweg nicht gewährleistet ist oder weil ggf. der Wirkstoff gekühlt aufbewahrt werden muss oder weil er ggf. empfindlich ist gegenüber Oxidation. Der Anwender eines medizinischen Pflasters muss dann aus einer vorher durch Entfernen der Verschlussfolie zu öffnenden, meist versiegelten und damit flüssigkeitsdichten Verpackung das wirkstoffhaltige Trägermaterial auf die Deckschicht eines transdermalen therapeutischen Systems (TTS) übertragen und es darauf fixieren. Der Anwender soll dabei aber wegen der Gefahr der Keimübertragung das wirkstoffhaltige Trägermaterial möglichst nicht mit den Fingern berühren und möglichst auch sonst keine zusätzlichen Hilfsmittel zum Übertragen verwenden.

Üblicherweise legt man daher die Deckschicht des TTS mit ihrer Unterseite auf die oberseitig geöffnete Verpackung eines wirkstoffhaltigen Trägermaterials und drückt dabei das Trägermaterial an die Deckschicht des TTS. Wenn das TTS danach wieder von der geöffneten Verpackung entfernt wird, bleibt das wirkstoffhaltige Trägermaterial an der Deckschicht des TTS haften und bildet zusammen mit diesem das TTS im eigentlichen Sinne, was aber nicht immer zuverlässig passiert. Oft oder zumindest mitunter kommt es vor, dass das Trägermaterial mit dem Wirkstoff einfach in der Verpackung liegen bleibt.

Unter anderem tritt dieses Problem beim Verfahren der lontophorese auf, die dann zum Einsatz kommt, wenn die passive transdermale Arzneimittelzufuhr für bestimmte Arten von Arzneistoffen nur sehr ineffizient ist. Insbesondere sind ionisierte Medikamente oft nicht in der Lage, passiv die Haut in therapeutisch wirksamer Menge zu durchdringen.

Der Prozess der lontophorese wurde ursprünglich von LeDuc im Jahr 1908 beschrieben, und noch früher in US-222,276 (1879) und US-486,902 (1892). Seitdem hat die lontophorese kommerziellen Einsatz bei der transdermalen Applikation von ionisch geladenen therapeutischen Wirkstoffmolekülen wie Pilocarpin, Lidocain, Dexamethason, Lidocain und Fentanyl gefunden.

Im Allgemeinen ist die lontophorese ein Applikationsverfahren, das auf dem Grundprinzip beruht, dass die Anwendung von elektrischem Strom externe Energie zur Verfügung stellt, mit deren Hilfe eine Erhöhung der Arzneimittelpermeabilität durch die Membranen der Haut möglich wird, was einen verbesserten Durchtritt von Wirkstoffionen durch die Haut bewirkt.

Wenn Ionen, die eine positive Ladung tragen (z. B. kationische Wirkstoffe), in oder unter der Anode eines iontophoretischen Systems platziert werden, dann wird auf diese Ionen - bei Anwendung von Strom - ein Impuls ausgeübt, der sie von der Anode weg und in Richtung des elektrischen Feldes hin zur Kathode schiebt, die in unmittelbarer Nähe der Haut angeordnet ist. Während dieses Vorgangs wird der Transport des kationischen Arzneimittels durch die Haut verbessert oder erleichtert.

Iontophorese kann mit verschiedenen Formen von pharmazeutischen Wirkstoffen durchgeführt werden, am günstigsten mit solchen, die eine elektrische Ladung besitzen und die somit im elektrischen Feld die Fähigkeit entwickeln, Barrieren (z.B. die Haut) zu überwinden.

Ein typisches iontophoretisches Arzneimittelabgabesystem umfasst ein elektrolytisches elektrisches System aus einer Anode und einer Kathode, die an verschiedenen - vorzugsweise benachbarten - Hautbereichen eines Patienten angeordnet sind, wobei jede Elektrode über einen Draht an eine externe Stromversorgung angeschlossen ist. In der Regel handelt es sich dabei um ein durch einen Mikroprozessor gesteuertes elektrisches Gerät. Solche Arten von Vorrichtungen sind bekannt, einschließlich der Systeme mit ausgesprochen einfacher Konstruktion (z.B. US 5,685,837 oder US 6,745,071) oder auch komplexere Systeme, die dem Fachmann grundsätzlich bekannt sind. Iontophoretische transdermale Systeme für Lidocain und Fentanyl wurden bereits erfolgreich in den USA am Markt eingeführt. Eine ganz besonders ausführliche Beschreibung eines Systems zur Abgabe von Arzneimittel mit Hilfe von lontophorese findet sich in der WO 2012/071175.

US 5,558,633 beschreibt, dass für die Abgabe von Medikamenten aus Flüssigkeit oder aus gelierten wässrigen Formulierungen lontophoresevorrichtungen besonders geeignet sind. Jedoch kann in solchen Vorrichtungen die iontophoretische Verabreichung von pharmazeutischen Wirkstoffen durch die Anwesenheit von "Hintergrund"-Elektrolyten (siehe, zum Beispiel, Luzardo-Alvarez, A., et al., Proceedings of the International Symposium on Controlled Release of bioactive Materials (2000), 27th Ed., S. 159 bis 160) stark beeinträchtigt sein. Hinsichtlich der Konstruktion von iontophoretischen Vorrichtungen besteht zudem ein Mangel an pharmazeutischen Gelen oder Flüssigkeiten, die sich selbst nicht als "Hintergrund"-Gegenionen störend auswirken.

Trotz immer noch bestehender diverser Mängel hat sich die lontophorese als Applikationsverfahren in allen den Fällen bewährt, in denen ein herkömmliches TTS nicht ausreichend ist, schnell die Verabreichung einer therapeutisch wirksamen Dosis eines solchen Wirkstoffs zu gewährleisten. Allerdings besitzt die lontophorese die inhärente Gefahr, dass Nebenwirkungen wie Hautreizungen, Hautrötungen, Brennen oder auch Haut Nekrose, insbesondere dann auftreten können, wenn die Stromstärke erhöht oder wenn die lontophorese über einen längeren Zeitraum eingesetzt wird. Andererseits kann eine Erhöhung der Stromstärke für die Verabreichung von höheren Dosen von therapeutischem Wirkstoff durchaus erwünscht sein, weil die Menge der Ionen, die transportiert werden, direkt proportional ist zu der Menge des fließenden Stroms pro Zeiteinheit.

In Anbetracht der vorstehenden Ausführungen war es daher die Aufgabe der vorliegenden Erfindung, ein Verfahren zu finden, mit dem ein wirkstoffhaltiges Trägermaterial zuverlässig aus seiner Verpackung entnommen und an der Deckschicht des TTS zwecks nachfolgender Applikation, sei es mit oder ohne Unterstützung durch lontophorese, fixiert werden kann, ohne dass dazu eine Berührung des wirkstoffhaltigern Trägermaterials mit der Hand oder ein zusätzliches Hilfsmittel zum Übertragen notwendig ist.

Gelöst wird diese Aufgabe durch ein TTS der eingangs genannten Gattung, umfassend eine Deckschicht und mindestens ein wirkstoffhaltiges Trägermaterial, wobei sich mindestens ein Halteelement zwischen wirkstoffhaltigem Trägermaterial und Deckschicht befindet, mit dem das wirkstoffhaltige Trägermaterial an der Deckschicht fixiert ist.

Vorzugsweise ist erfindungsgemäß das Halteelement als ein länglich geformtes Segment eines Klettbandes ausgebildet.

Unter einem Klettband ist anmeldungsgemäß ein textiles, beliebig oft zu lösendes Verschlussmittel zu verstehen, das auf dem Prinzip von Klettfrüchten beruht. Die bionische Umsetzung besteht in der typischen Form aus zwei gewebten Faserstreifen, von denen der eine flexible Widerhäkchen oder Pilzköpfe, der andere hingegen Schlaufen aufweist. Zusammengepresst ergeben sie einen mehr oder weniger belastungsfähigen, auf jeden Fall aber reversiblen Schnellverschluss. Gewebte Klettbänder bestehen aus Polyamid-, Polyester- oder Polyolefinfasern. Bei den Hakenbändern werden die Haken während des Webens oder später eingearbeitet. Klettbänder und Druckverschlüsse können auch mit Haftklebstoffen auf der Rückseite selbstklebend beschichtet werden.

Erfindungsgemäß bildet das Klettband, das an der Deckschicht des TTS angeordnet ist, die Hakenseite, während das wirkstoffhaltige Trägermaterial die Funktion der Schlaufenseite übernimmt. Durch Andrücken des Halteelements, das an der Deckschicht des TTS angebracht ist, auf die Oberseite der nach oben hin durch vorheriges Entfernen der Verschlussfolie geöffneten Verpackung, in der sich das wirkstoffhaltige Trägermaterial befindet, wird erfindungsgemäß bewirkt, dass sich das Klettband mit dem Trägermaterial verhakt, beim Abnehmen des TTS von der Verpackung am Halteelement des TTS haftet und nicht in der Verpackung zurückbleibt, wenn diese danach wieder entfernt wird.

Als Deckschicht wird anmeldungsgemäß ein im wesentlichen selbsttragendes Flächengebilde bezeichnet, das die Abdeckung der Stelle auf der Haut bewirkt, an der die transdermale Abgabe von pharmazeutischem Wirkstoff an den Organismus stattfindet. Wenn die Befestigung des TTS auf der Haut nicht über ein haftklebend ausgerüstetes Überpflaster erfolgt, dann hat die Deckschicht zweckmäßigerweise auch die Funktion, das System auf der Haut zu fixieren, was durch eine flächig haftklebende oder musterförmig haftklebende Ausgestaltung der in die Richtung der Haut weisenden Seite der Deckschicht geschieht, wobei die Fläche oder die Flächen für das wirkstoffhaltiges Trägermaterial oder die wirkstoffhaltigen Trägermaterialien zumindest frei von Haftkleber sind.

In einer weiteren Ausgestaltungsform der Erfindung handelt es sich bei der Deckschicht um eine okklusive Deckschicht. Unter einer okklusiven Deckschicht ist insbesondere eine Barriereschicht für Flüssigkeiten und Wasserdampf zu verstehen, mit der verhindert wird, dass Flüssigkeit oder Wasserdampf durch die Deckschicht hindurch nach außen entweicht, wodurch das System austrocknen würde, falls es sich um die Abgabe von flüssigem oder in Flüssigkeit gelöstem pharmazeutischen Wirkstoff handelt.. Die okklusive Deckschicht kann erfindungsgemäß aus einer Folie aus Kunststoff wie Polyethylen oder Polypropylen oder Polyester oder Polyurethan oder Polyamid bestehen, deren wesentliche Eigenschaft darin zu sehen ist, dass sie eine ausreichend zuverlässige Sperre für Wasser, Wasserdampf oder mit Wasser vermischten organischen Lösemitteln darstellt. Geeignete Folien aus den genannten Kunststoffen haben eine Dicke im Bereich von 5 bis 300 µm, vorzugsweise von 10 bis 200 µm, besonders bevorzugt von 12 bis 150 µm.

Die okklusive Deckschicht kann zusätzlich zur Verstärkung noch eine nicht okklusive Trägerschicht enthalten oder mit einer nicht okklusiven Trägerschicht verbunden sein. Als nicht okklusive Trägerschicht kann ein textiles Gewebe geeignet sein oder ein Vlies oder Filzmaterial oder sonstiges cellulosehaltiges Material. Die Verbindung der nicht okklusiven Trägerschicht mit der okklusiven Deckschicht kann durch Laminieren unter Druck oder durch Extrudieren oder durch Verkleben mit einem geeigneten Klebstoff erfolgen. Geeignete Kleber können aus Polyisopren oder Polyisobutylen oder Polyacrylester oder auch aus Polysiloxancopolymeren bestehen.

Auch die okklusive Deckschicht kann zusätzlich an ihrer Unterseite, das ist die in Richtung zur Haut weisende Seite, mit einer selbstklebenden Schicht versehen sein. Als selbstklebende Materialien eignen sich die vorher genannten Kleber, vorzugsweise Acrylkleber.

In einer weiteren Ausführungsform ist das wirkstoffhaltige Trägermaterial ein mit pulverförmigem Wirkstoff bestäubtes oder ein flüssigkeitsgetränktes Trägermaterial oder ein Hydrogel. Als Flüssigkeit kann erfindungsgemäß eine Lösung eines Wirkstoffes in einem Lösemittel, vorzugsweise in einem wässrigen Lösemittel, gelten oder aber auch eine Dispersion oder eine Emulsion von Wirkstoff in einem geeigneten Dispergier- oder Emulgiermittel. Als Trägermaterialien kommen dabei vliesartige Materialien oder textile gewebte Materialien oder Gewirke oder schwammförmige Materialien vorteilhaft zum Einsatz oder ggf. auch gelbildende Polymere.

Erfindungsgemäß bevorzugt ist die Form des länglich geformten Klettbandes der Form des wirkstoffhaltigen Trägermaterials angepasst. Wenn das wirkstoffhaltige Trägermaterial eine rechteckige Form besitzt, dann entspricht die Länge des länglich geformten Klettbandes vorzugsweise der Länge einer der Seiten des rechteckig geformten, wirkstoffhaltigen Trägermaterials. Wenn das wirkstoffhaltige Trägermaterial eine runde oder ovale Form besitzt, dann ist die Form des länglich geformten Klettbandes vorzugsweise die eines gebogenen Kreissegments mit einem Radius entsprechend dem Radius des runden oder ovalen wirkstoffhaltigen Trägermaterials.

Erfindungsgemäß geeignete Klettbänder sind selbstklebend, d.h. sie haften mit ihrer selbstklebenden Rückseite an der Deckschicht. Es ist aber auch möglich, dass ein Klettband mit einem geeigneten Kleber, z.B. einem Acrylkleber oder einem Zweikomponentenkleber oder einem Heißschmelzkleber an der Deckschicht befestigt wird.

Die Breite des länglich geformten Klettbandes kann in weiten Grenzen variieren. Das Klettband muss nicht die gesamte Fläche bedecken, über die sich das wirkstoffhaltige Trägermaterial im TTS erstreckt, sondern es ist ausreichend, wenn das länglich geformte Klettband, unabhängig von der Geometrie des wirkstoffhaltigen Trägermaterials, nur einen Teil der Fläche bedeckt. Möglich ist dabei auch, dass sich das Klettband nur in den seitenrandnahen Bereichen befindet oder dass es als umlaufender Streifen ausgebildet ist mit einer Breite im Bereich von 2 bis 15 mm, vorzugsweise von 3 bis 10 mm.

Erfindungsgemäß kann es sich auch um mehrere Klettbänder handeln, die z.B. an gegenüberliegenden Seiten der Fläche angeordnet sind, an der das wirkstoffhaltige Trägermaterial an dem TTS befestigt ist oder werden soll.

Die Erfindung betrifft auch ein Verfahren zum Befestigen eines wirkstoffhaltigen Trägermaterials an einer Deckschicht eines TTS in Anwesenheit von Klettbandsegmenten. Dabei wird das wirkstoffhaltige Trägermaterial in einer nach oben hin durch vorheriges abziehen einer Verschlussfolie geöffneten Verpackung an die okklusive Deckschicht und die dort angeordneten Klettbandsegmente angenähert. Dann wird die Verpackung zusammen mit dem darin befindlichen wirkstoffhaltigen Trägermaterial durch Pressen an die Klettbandsegmente an der okklusiven Deckschicht angedrückt. Danach kann die Verpackung losgelöst und entfernt werden, wobei das wirkstoffhaltige Trägermaterial an den Klettbandsegmenten und an der okklusiven Deckschicht fixiert bleibt.

Die Erfindung betrifft ferner die Verwendung eines Klettbandes als Bestandteil eines transdermalen therapeutischen Systems (TTS) oder als Bestandteil eines iontophoretischen transdermalen therapeutischen Systems.

Die Erfindung umfasst ferner die Verwendung eines Klettbandes in einem Verfahren zur transdermalen oder iontophoretischen Verabreichung von kationischen Wirkstoffen an Patienten, die eine Behandlung mit solchen Wirkstoffen erfordern.

Das System der vorliegenden Erfindung ist insbesondere in Verbindung mit therapeutischen Wirkstoffen, die eine kationische Struktur besitzen, insbesondere Wirkstoffe, die Amino- oder Iminogruppen in ihrem Molekül aufweisen, besonders nützlich.

Dementsprechend ist die vorliegende Erfindung geeignet für die transdermale, insbesondere die iontophoretische Verabreichung von Analgetika wie Fentanyl oder Morphin, Antiemetika wie Granisetron oder andere auf das zentrale Nervensystem wirkende Arzneistoffe wie Rivastigmin oder Galantamin.

Wenn das erfindungsgemäße System zur transdermalen Verabreichung von solchen Wirkstoffen verwendet wird, dient das flüssigkeitsgetränkte Trägermaterial als eine Matrix oder ein Reservoir, aus dem die kationischen Wirkstoffe an die Haut abgegeben werden und dann die Haut durchdringen, entweder passiv oder unterstützt durch lontophorese.

Unter Wirkstoffen mit kationischer Struktur sind allgemein Wirkstoffe zu verstehen, die als positiv geladene Ionen (Kationen) vorliegen oder die zur Bildung positiv positiv geladener Ionen in wässerigen Medien befähigt sind. Zum Beispiel haben viele biologisch aktive Mittel funktionelle Gruppen, die in wässrigem Medium jederzeit in ein positiv geladenes Ion und ein Gegenion dissoziieren, z.B. lösliche Salze basischer Wirkstoffe.

Der Ausdruck "Wirkstoffe" umfasst insbesondere therapeutisch aktive Mittel, pharmakologisch aktive Mittel und andere Mittel mit vorteilhaften Wirkungen, wenn man sie einem Menschen oder einem Tier verabreicht.

Im Allgemeinen bezeichnet der Begriff "Wirkstoffe" pharmazeutische Wirkstoffe oder Arzneimittel, also therapeutische Wirkstoffe. Der Ausdruck "Wirkstoffe" umfasst weiter auch Mittel zur Verwendung in der Veterinärmedizin.

Die vorliegende Erfindung ist besonders geeignet für die transdermale, insbesondere die iontophoretische, Verabreichung von Wirkstoffen wie
- Opioid-Agonisten, einschließlich Schmerzmittel wie Fentanyl, Sufentanyl, Morphin, Morphin-Derivate wie Codein oder die Heroin, Dihydrocodein, Hydromorphin, Oxycodon, Hydrocodon, Pethidin, Loperamid, Diphenoxylat, Methadon, Tramadol oder Tilidin;
- Opioid-Antagonisten wie Naloxon, Naltrexon;
- Gemischte Opiat-Agonisten/Antagonisten, wie Buprenorphin, Pentazocin, Nalbuphin;
- Antiemetika einschließlich 5-HT₃-Rezeptor-Antagonisten wie Granisetron, Lerisetron, Ondansetron, Dolasetron, Metoclopramid sowie antidopaminerge Medikamente wie Domperidon, sowie H1-Rezeptor-Antagonisten wie beispielsweise Promethazin oder Meclozin sowie Muscarin-Antagonisten wie Scopolamin;
- Arzneimittelverbindungen, die auf das zentrale Nervensystem wirken, wie Rivastigmin, Galantamin, Tacrin, Donepezil, sowie Pramipexol, Adrenalin, Dopamin, Ropinirol, Nikotin, Fluphenazin, Chlorpromazin, Benzodiazepine, Monoamin-Wiederaufnahmehemmer wie Amitriptylin, Antidepressiva wie Mianserin;
- Alkaloide wie Ergotamin, Dihydroergotamin, Methysergid oder Lisurid, Belladonna-Alkaloide;
- Peptide, insbesondere Peptidhormone wie Insulin und Oxytocin oder Blutgerinnungsfaktoren und Wachstumshormone;
- kationisch aktive Indol-Verbindungen wie N-Dimethyltryptamin oder Psilocin;
- Lokalanästhetika wie Lidocain, Buprivacain, Artikain, Procain;
- Gastrointestinal wirkende Therapeutika, wie Carnitinchlorid oder Metoclopramid;
- Muskelrelaxantien wie Vancuroniumbromid;
- Antibiotika wie Tetracyclin, Tetracyclinbasierte-Zubereitungen, Kanamycin, Kanamycinbasierte-Zubereitungen, Gentamycin, Gentamycinbasierte -Zubereitungen oder Chinin;
- Gewichtsreduktionsmittel wie Fenfluramin oder Ephedrin;
- Antidiabetika wie Metformin;
- Inhibitoren der Thrombozytenaggregation, z. B. Ticlopidin oder Clopidogrel;
- Antiarrhythmika wie Chinidin oder Lidocain;
- Herz- oder Herz-Kreislaufmittel wie Dopamin, Noradrenalin, Methoxamin, Adrenalin, Verapamil, Diltiazem, Propranolol, Clonidin, Tolazolin;
- Sympathomimetika wie Salbutamol oder Terbutalin;
- Antihistaminika wie Clemastin, Cetirizin oder Chlorphenoxamin.

In einer bevorzugten Ausführungsform ist der Wirkstoff aus der Gruppe der kationischen Indol-Verbindungen, besonders aus der Gruppe der kationischen Indol-Verbindungen, N-dimethyltryptamin und Psilocin, wobei die Gruppe auch die pharmazeutisch geeigneten Salze dieser kationisch aktiven Indol-Verbindungen mit umfasst.

Die vorstehend genannten kationischen Wirkstoffe können auch in Form von pharmazeutisch geeigneten Salzen vorliegen. Beispiele für pharmazeutisch geeignete Salze umfassen, Chlorid-, Bromid-, Jodid-, Schwefel-, Phosphat-, Lactat-, Citrat-, Tartrat-, Salicylat-, Succinat-, Maleat-, Gluconat-, Mesylat-, Laurat-, Dodecylat-, Myristat-, Palmitate-,Stearatsalze sind jedoch nicht beschränkt auf diese.

Die Intensität des Stroms während der lontophorese sollte idealerweise den Wert von 600 µA/cm² nicht überschreiten, um das brennende Gefühl oder Verbrennungen der Haut zu vermeiden. Die Ausgangsspannung liegt im Allgemeinen im Bereich von 0,5 bis 10 V, je nach dem Widerstand zwischen den beiden Elektroden und dem Zwischenbereich der Haut, der normalerweise 50 kΩ oder mehr betragen kann.

In einer weiteren Ausführungsformen enthält das flüssigkeitsgetränkte Trägermaterial den kationischen Wirkstoff oder ein Salz davon in einer Menge im Bereich von 0,1 bis 20 Gew.-%., vorzugsweise von 0,2 bis 10 Gew.-%., besonders bevorzugt von 2 bis 10 Gew.-%., ganz besonders bevorzugt von 3 bis 5 Gew.-%., bezogen auf das Gesamtgewicht an Flüssigkeit in dem Trägermaterial.

Das flüssigkeitsgetränkte Trägermaterial gemäß der vorliegenden Erfindung enthält als Flüssigkeit vorzugsweise Wasser oder ein wässriges Lösungsmittelgemisch. Vorzugsweise ist der Anteil an Wasser in dem Lösungsmittelgemisch mindestens 15 Gew.-%, besonders bevorzugt mindestens 40 Gew.-%, bezogen auf das Gesamtgewicht der Flüssigkeit.

Gemäß einer weiteren Ausführungsform der Erfindung liegt der Wassergehalt oder der Anteil des Lösungsmittelgemisches im Bereich von 80 bis 99 Gew.-%.

Der Begriff "wässriges Lösemittelgemisch" umfasst im Allgemeinen flüssige Mischungen, die Wasser und mindestens ein weiteres Lösungsmittel enthalten, das im allgemeinen ein polares, mit Wasser mischbares organisches Lösungsmittel ist, wie z.B. Alkohole wie Ethanol, Isopropanol oder Glycerin.

Die vorliegende Erfindung umfasst auch Anwendungen, bei denen ein kationischer Wirkstoff in Kombination mit mindestens einem weiteren Wirkstoff eingesetzt wird, der ausgewählt ist aus der Gruppe bestehend aus aktiven Mitteln mit einer neutralen Ladung und der sogar auch anionische Wirkstoffe umfassen kann.

Im allgemeinen werden solche Wirkstoffe in dem System der vorliegenden Erfindung eingesetzt, die zur Durchdringung der Haut durch passive Diffusion befähigt sind oder die für die iontophoretische Hautpermeation geeignet sind.

In einer weiteren Ausführungsform kann das flüssigkeitsgetränkte Trägermaterial eine Hydrogel-Zusammensetzung sein, wobei zusätzliche gelbildende Polymere anwesend sind, die aus der Gruppe bestehend aus Polyacrylaten und Zellulosederivaten, wie Hydroxypropylmethylcellulose, Hydroxypropylcellulose oder Hydroxyethylcellulose, ausgewählt sein können.

Die Verwendung von Hydrogel-Zubereitungen bei der lontophorese ist besonders vorteilhaft, weil in diesem Fall die Ionenstärke durch Veränderung des Anteils des Wassers innerhalb des Hydrogels eingestellt werden kann. Somit kann die Ionenstärke leicht eingestellt werden, um die Wirksamkeit des iontophoretischen Prozesses in jedem speziellen Fall zu optimieren.

In einer weiteren Ausführungsform weist die Flüssigkeit in dem flüssigkeitsgetränkten Trägermaterial einen pH-Wert im Bereich von 3 bis 8, vorzugsweise von 5,5 bis 7 besonders bevorzugt von etwa 6 auf.

Im allgemeinen ist es bevorzugt, den pH-Wert so einzustellen, dass er sich nicht wesentlich von dem pH-Wert der Haut unterscheidet, wenn das TTS auf die Haut aufgebracht wird. In einer weiteren Ausführungsform ändert sich der pH-Wert der Haut zu ± 4,0 oder weniger, ungefähr ± 3,5 oder weniger, ungefähr ± 3,0 oder weniger, ungefähr ± 2,5 oder weniger, ungefähr ±2,0 oder weniger, etwa ± 1,5 oder weniger, ungefähr ± 1,0 oder weniger oder etwa ± 0,5 oder weniger. Substanzen und Puffer für die Einstellung des pH-Wertes sind dem Fachmann bekannt.

Das flüssigkeitsgetränkte Trägermaterial kann optional weitere Additive enthalten, wobei die Additive ausgewählt sein können aus der Gruppe Lösungsvermittler, Hautdurchdringungsverstärker, Konservierungsmittel und antimikrobielle Mittel.

In diesem Zusammenhang bedeutet der Begriff "Löslichkeitsverbesserer" allgemein Verbindungen, die zur Erhöhung der Löslichkeit des kationisch aktiven Mittels in der Flüssigkeit beitragen können. Dies kann entweder durch Modulieren der möglichen Wechselwirkungen zwischen dem kationischen Wirkstoff und den anderen in der Flüssigkeit enthaltenen Komponenten oder durch den zusätzlichen Einbau geeigneter Hilfsstoffe erreicht werden.

Alternativ kann die Löslichkeit des Wirkstoffes durch Veränderung der Kristallmodifikation erreicht werden.

Beispiele für Lösungsvermittler umfassen Wasser, Diole, wie Propylenglykol und Glycerin, Monoalkohole wie Ethanol, Propanol und höhere Alkohole, Dimethylsulfoxid (DMSO), Dimethylformamid, N,N-Dimethylacetamid, N-substituierte Alkyl-Azacycloalkyl-2-one.

Ferner sind unter dem Begriff "Hautpermeationsverstärker" insbesondere Verbindungen zu verstehen, die eine Erhöhung der Permeabilität der Haut für einen Wirkstoff enthalten, insbesondere für einen kationischen Wirkstoff. Aufgrund dieser Erhöhung der Hautdurchlässigkeit wird die Rate, mit der der Wirkstoff die Haut durchdringt und in die Blutzirkulation eindringt, verstärkt.

Beispiele für Permeationsverstärker umfassen Dimethylsulfoxid (DMSO), N,N-Dimethylacetamid (DMA), Decylmethylsulfoxid (C10 MSO), Polyethylenglykolmonolaurat (PEGML), Propylenglykol (PG), Propylenglycolmonolaurat (PGML), Glycerinmonolaurat (GML), Lecithin, die 1-substituierten Alkyl-Azacycloalkyl-2-one, insbesondere Indodecyl-cylazacycloheptan-2-on, Alkohole und dergleichen.

Der Permeationsverstärker kann auch aus pflanzlichen Ölen ausgewählt werden, z. B. Safloröl, Baumwollsamenöl oder Maisöl.

Kombinationen, die zwei oder mehr verschiedene Permeationsverstärker enthalten, können ebenfalls verwendet werden.

Ferner sind unter dem Begriff "antimikrobielles Mittel" allgemein Mittel zu verstehen, die zur Verhinderung des Wachstums von Mikroben in einer pharmazeutischen Zubereitung, insbesondere in der Flüssigkeit des flüssigkeitsgetränkten Trägermaterials gemäß der vorliegenden Erfindung, geeignet sind.

Beispiele für geeignete antimikrobielle Mittel umfassen Salze von Chlorhexidin, wie lodpropynyl Butylcarbamate, Diazolidinyl-Harnstoff, Chlorhexidindigluconat, Chlorhexidinacetat, Chlorhexidinisothionat oder Chlorhexidin-Hydrochlorid. Andere kationische antimikrobielle Mittel können ebenfalls verwendet werden, wie beispielsweise Benzalkoniumchlorid, Benzethoniumchlorid, Triclocarbon, Polyhexamethylenbiguanid, Cetylpyridiniumchlorid, Methylbenzethoniumchlorid.

Andere antimikrobielle Mittel umfassen halogenierte phenolische Verbindungen, wie 2,4, 4'-Trichlor-2-hydroxydiphenylether (Triclosan), parachlorometa Xylenol (PCMX), Methylpara-hydroxybenzoat, und kurzkettige Alkohole, wie Ethanol, Propanol und dergleichen. Vorzugsweise ist die Gesamtkonzentration der antimikrobiellen Mittel im Bereich von 0,01 bis 2 Gew.-%., bezogen auf das Gesamtgewicht der Flüssigkeit, in der es enthalten ist.

Geeignete Trägermaterialien können Faserlagen, Gewebe, Gewirke, Schwämme, Schwammtuch, Gewirke-Vlies oder Gewebe-Filze oder filzartige Materialien usw. sein.

Die vorliegende Erfindung betrifft ferner die Verwendung des oben beschriebenen Systems als ein integraler Bestandteil eines iontophoretischen Pflasters, vorzugsweise als anodisches Reservoir des Pflasters.

### Beispiel

Im folgenden wird die Erfindung und ihre Wirksamkeit beispielhaft anhand der beigefügten Zeichnungen veranschaulicht.

Fig. 1 zeigt schematisch ein erfindungsgemäßes TTS in der Ansicht von unten, also von der Hautseite.

Fig. 2 zeigt schematisch ein erfindungsgemäßes TTS in der Ansicht von oben, also von der von der Haut abgewandten Seite.

Fig. 3a, 3b, 3c und 3d zeigen schematisch in Teilschritten wie das wirkstoffhaltige Trägermaterial aus seiner getrennt gelagerten Verpackung entnommen und an der Deckschicht des TTS fixiert wird.

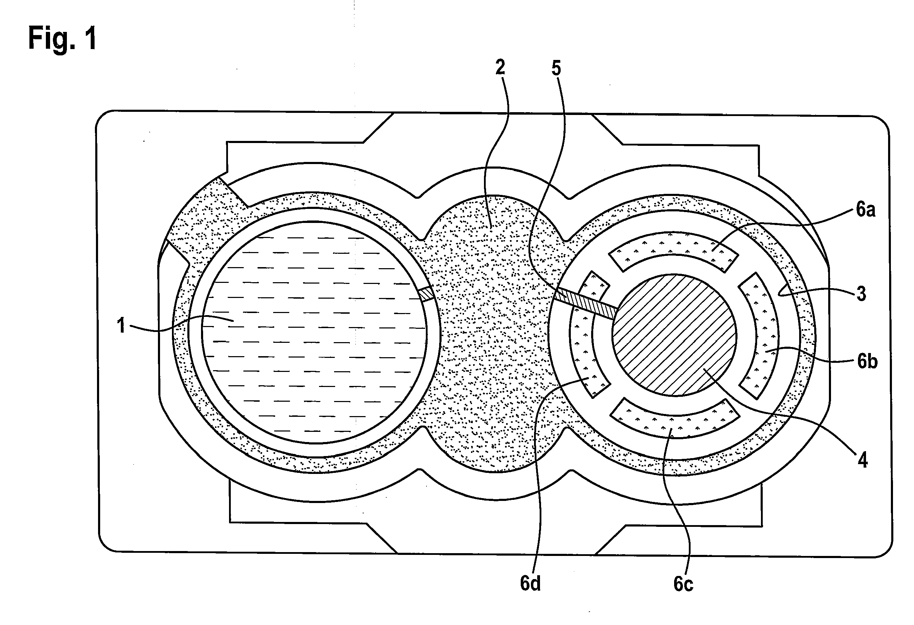

In Fig. 1 ist das wirkstoffhaltige Trägermaterial 1 zu erkennen, das eine runde Form besitzt uns das an der linken Fläche des TTS bereits in Position gebracht ist, während eine Abdeckfolie 2 mit einer Öffnung 3 an der rechten Seite den Blick auf die Elektrode 4, die elektrische Zuleitung 5 und vier gebogen geformte Klettbänder 6a, 6b, 6c und 6d ermöglicht. Auf die Klettbänder 6a, 6b, 6c und 6d wird dann im nächsten Schritt ein weiteres in der Abbildung nicht dargestelltes wirkstoffhaltiges Trägermaterial aufgepresst und mit den Klettbändern 6a, 6b, 6c und 6d ortsfest fixiert.

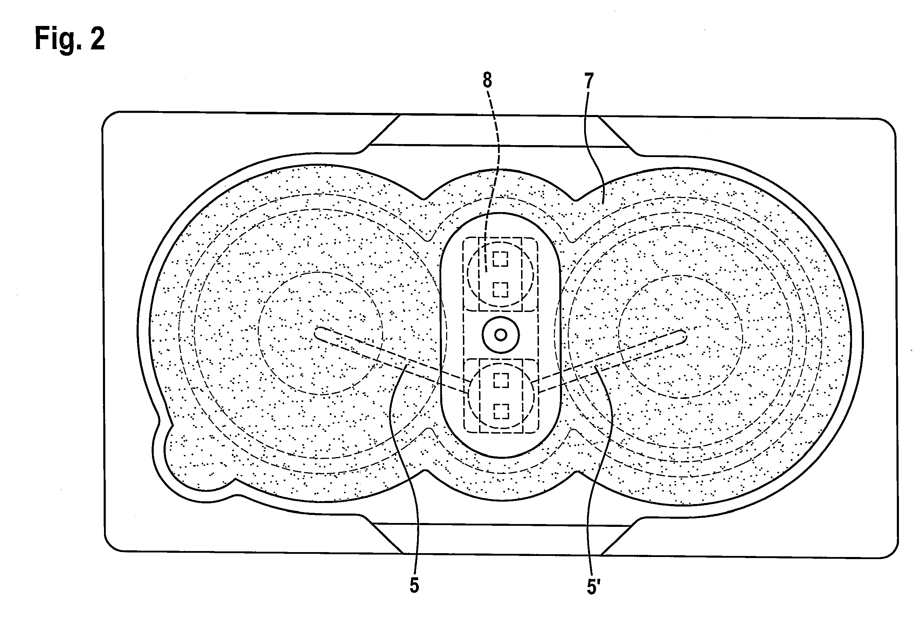

Fig. 2 zeigt im wesentlichen die Deckschicht 7, durch die hindurch die linke Stromzufuhr 5 und die rechte Stromzufuhr 5' erkennbar sind. Mittig in der Darstellung ist die Einheit zur Stromversorgung 8 mit Batterie und elektronischer Regelung zu erkennen.

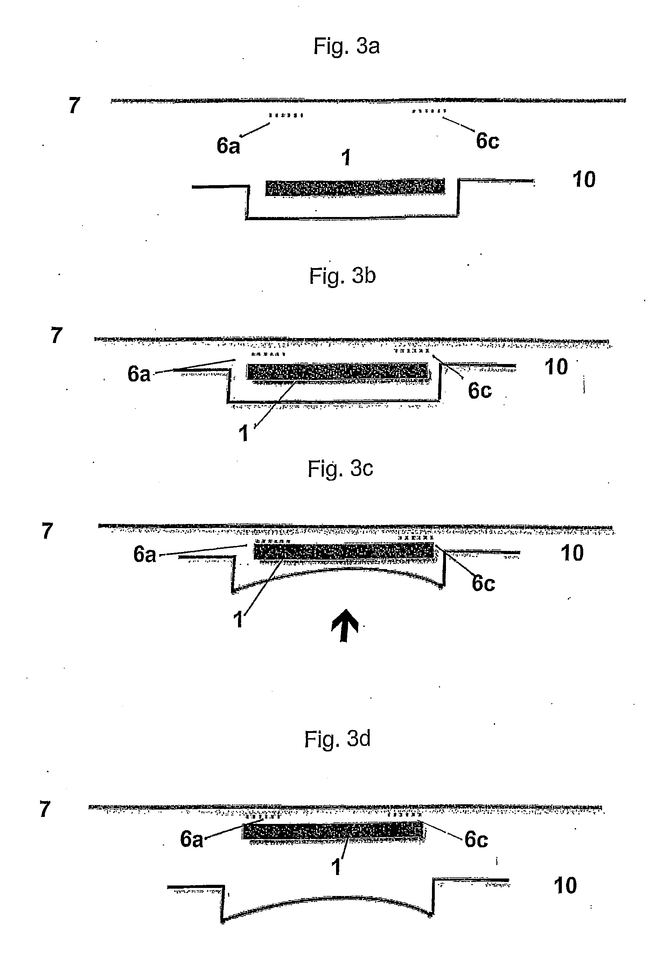

Fig. 3a zeigt im oberen Bereich die Deckschicht 7, an der zwei Klettbandsegmente 6a und 6c angeordnet sind, und im unteren Bereich das wirkstoffhaltige Trägermaterial 1 das in einer nach oben hin durch vorheriges, nicht dargestelltes Abziehen einer Verschlussfolie geöffneten Verpackung 10 lagert.

Fig. 3b zeigt wie das wirkstoffhaltige Trägermaterial 1 noch in der geöffneten Verpackung 10 und zusammen mit dieser an die Deckschicht 7 und die Klettbandsegmente 6a und 6c angenähert ist.

Fig. 3c zeigt wie das wirkstoffhaltige Trägermaterial 1 zusammen mit der Verpackung 10 von unten in Pfeilrichtung an die Klettbandsegmente 6a und 6c und an die Deckschicht 7 angepresst wird.

Fig. 3d zeigt, wie die Verpackung 10 nach unten entfernt wird, wobei das wirkstoffhaltige Trägermaterial 1 an den Klettbandsegmenten 6a und 6c und damit an der Deckschicht 7 fixiert bleibt.

## Patentansprüche

1. Transdermales therapeutisches System (TTS) für die Abgabe von pharmazeutischen Wirkstoffen umfassend eine Deckschicht und mindestens ein wirkstoffhaltiges Trägermaterial, wobei sich mindestens ein Halteelement zwischen wirkstoffhaltigem Trägermaterial und Deckschicht befindet, mit dem das wirkstoffhaltige Trägermaterial an der Deckschicht fixiert ist.

2. TTS nach Anspruch 1, **dadurch gekennzeichnet, dass** das Halteelement die Hakenseite eines länglich geformten Segments eines Klettbandes ist.

3. TTS nach Anspruch 1 oder 2, **dadurch gekennzeichnet, dass** die Befestigung des TTS auf der Haut über ein haftklebend ausgerüstetes Überpflaster erfolgt oder über eine flächig haftklebende oder musterförmig haftklebende Ausgestaltung der in Richtung zur Haut weisenden Seite der Deckschicht, wobei die Fläche oder die Flächen für das wirkstoffhaltiges Trägermaterial oder die wirkstoffhaltigen Trägermaterialien zumindest frei von Haftkleber sind.

4. TTS nach einem oder nach mehreren der Ansprüche 1 bis 3, **dadurch gekennzeichnet, dass** die Deckschicht eine okklusive Deckschicht ist, die eine Barriereschicht für Flüssigkeiten und Wasserdampf ist.

5. TTS nach Anspruch 4, **dadurch gekennzeichnet, dass** die okklusive Deckschicht aus einer Folie aus Kunststoff wie Polyethylen oder Polypropylen oder Polyester oder Polyurethan oder Polyamid besteht, und eine Dicke im Bereich von 5 bis 300 µm, vorzugsweise von 10 bis 200 µm, besonders bevorzugt von 12 bis 150 µm besitzt.

6. TTS nach einem oder nach mehreren der Ansprüche 1 bis 5, **dadurch gekennzeichnet, dass** die Form des länglich geformten Klettbandes der Form des wirkstoffhaltigen Trägermaterials angepasst ist.

7. TTS nach einem oder nach mehreren der Ansprüche 1 bis 6, **dadurch gekennzeichnet, dass** das wirkstoffhaltige Trägermaterial eine runde oder ovale Form besitzt und dass das länglich geformte Klettband die Form eines gebogenen Kreissegments hat mit einem Radius entsprechend dem Radius des runden oder ovalen flüssigkeitsgetränkten Trägermaterials.

8. TTS nach einem oder nach mehreren der Ansprüche 1 bis 7, **dadurch gekennzeichnet, dass** die Breite des länglich geformten Klettbandes variieren kann, wobei das Klettband nicht die gesamte Fläche der Deckschicht bedeckt, über die sich das wirkstoffhaltige Trägermaterial im TTS erstreckt.

9. TTS nach Anspruch 8, **dadurch gekennzeichnet, dass** das länglich geformte Klettband nur einen Teil der Fläche bedeckt, wobei es vorzugsweise in den seitenrandnahen Bereichen der Fläche angeordnet ist, über die sich das wirkstoffhaltige Trägermaterial im TTS erstreckt..

10. TTS nach einem oder nach mehreren der Ansprüche 1 bis 9, **dadurch gekennzeichnet, dass** es mehrere Klettbänder umfasst, die an gegenüberliegenden Seiten der Fläche angeordnet sind, über die sich das wirkstoffhaltige Trägermaterial im TTS erstreckt.

11. TTS nach einem oder nach mehreren der Ansprüche 1 bis 10, **dadurch gekennzeichnet, dass** das wirkstoffhaltige Trägermaterial ein flüssigkeitsgetränktes Trägermaterial ist, wobei das Trägermaterial ein Vlies, ein textiles Material, ein Gewebe, ein Gewirke oder ein schwammförmiges Material oder Schwammtuch oder ein gelbildendes Polymer ist.

12. Verfahren zum Befestigen eines wirkstoffhaltigen Trägermaterials an einer Deckschicht eines TTS in Anwesenheit von Klettbandsegmenten wobei das wirkstoffhaltige Trägermaterial in einer nach oben hin durch vorheriges Abziehen einer Verschlussfolie geöffneten Verpackung an die Deckschicht und die dort angeordneten Klettbandsegmente angenähert wird, wobei dann die Verpackung zusammen mit dem darin befindlichen wirkstoffhaltigen Trägermaterial durch Pressen an die Klettbandsegmente an der Deckschicht angedrückt wird, wobei danach die Verpackung losgelöst und entfernt wird und wobei das wirkstoffhaltige Trägermaterial an den Klettbandsegmenten und an der Deckschicht fixiert bleibt.

13. Verwendung eines Klettbandes in einem Verfahren zur transdermalen oder iontophoretischen Verabreichung von therapeutischen Wirkstoffen an Patienten, die eine Behandlung mit solchen Wirkstoffen erfordern, zum Befestigen eines wirkstoffhaltigen Trägermaterials an einer okklusiven Deckschicht.

14. Verwendung nach Anspruch 13, **dadurch gekennzeichnet, dass** die therapeutischen Wirkstoffe, die eine kationische Struktur besitzen, solche Wirkstoffe sind, die Amino- oder Iminogruppen in ihrem Molekül aufweisen.

15. Verwendung nach Anspruch 13 oder 14 für die transdermale, iontophoretische Verabreichung von Analgetika wie Fentanyl oder Morphin, Antiemetika wie Granisetron oder andere auf das zentrale Nervensystem wirkende Arzneistoffe wie Rivastigmin oder Galantamin oder Peptide, insbesondere Peptidhormone wie Insulin und Oxytocin oder Blutgerinnungsfaktoren und Wachstumshormone.

16. Verwendung nach einem der Ansprüche 13 bis 15, **dadurch gekennzeichnet, dass** das wirkstoffhaltige Trägermaterial ein flüssigkeitsgetränktes Trägermaterial ist, das als Matrix oder als Reservoir dient, aus dem die therapeutischen Wirkstoffe an die Haut abgegeben werden und dann die Haut durchdringen, entweder passiv oder unterstützt durch lontophorese.
